# EUROPÄISCHE PATENTSCHRIFT

(11) **EP 0 939 833 B1**
(45) Veröffentlichungstag und Bekanntmachung des Hinweises auf die Patenterteilung: **16.01.2002**
(21) Anmeldenummer: 97922940.8
(22) Anmeldetag: 02.05.1997
(51) Int. Cl.: C12Q 1/68

(54) **VERWENDUNG EINER GENVERÄNDERUNG IM GEN FÜR DIE HUMANE G-PROTEIN beta3-UNTEREINHEIT ZUR DIAGNOSTIK VON ERKRANKUNGEN**
USE OF A MUTATION IN THE GENE FOR HUMAN G-PROTEIN beta 3 SUB-UNIT FOR DIAGNOSING ILLNESSES
UTILISATION D'UNE MUTATION DANS LE GENE RELATIF A LA SOUS-UNITE DE LA PROTEINE G beta 3 HUMAINE EN VUE DE DIAGNOSTIQUER DES MALADIES

(30) Priorität: 14.05.1996 DE 19619362
(43) Veröffentlichungstag der Anmeldung: 08.09.1999
(73) Patentinhaber: Siffert, Winfried, 45147 Essen (DE)
(72) Erfinder: Siffert, Winfried, 45147 Essen (DE)
(74) Vertreter: Schüll, Gottfried, Dipl.-Phys.
(86) Internationale Anmeldenummer: EP9702250
(87) Internationale Veröffentlichungsnummer: WO9743442

(56) Entgegenhaltungen:
- WO-A-00/15785
- WO-A-98/11212
- COTTON R G H: "CURRENT METHODS OF MUTATION DETECTION" MUTATION RESEARCH, Bd. 285, 1.Januar 1993, Seiten 125-144, XP000443992
- LEVINE ET AL.: "MOLECULAR CLONING OF BETA3 SUBUNIT, A THIRD FORM OF THE G PROTEIN BETA-SUBUNIT POLYPEPTIDE" PROC. NATL. ACAD. SCI. , Bd. 87, 1990, Seiten 2329-2333, XP002041163 in der Anmeldung erwähnt
- SIFFERT ET AL.: "ENHANCED G PROTEIN ACTIVATION IN IMMORTALIZED LYMPHOBLASTS FROM PATIENTS WITH ESSENTIAL HYPERTENSION" J. CLIN. INVEST., Bd. 96, 1995, Seiten 759-766, XP002041164
- T.Iiri & H.R.Bourne, Nature Genetics, vol.18, pages 8-10, (January 1998)
- W.Siffert et al, Nature Genetics, vol.18, pages 45-48, (January 1998)

### SEQUENZPROTOKOLL

(1) ALGEMEINE INFORMATION:
   (i) ANMELDER:
      (A) NAME: BASF Aktiengesellschaft
      (B) STRASSE: Carl-Bosch-Strasse 38
      (C) ORT: Ludwigshafen
      (E) LAND: Bundesrepublik Deutschland

      (F) POSTLEITZAHL: D-67056
      (G) TELEPHON: 0621/6048526
      (H) TELEFAX: 0621/6043123
      (I) TELEX: 1762175170
   (ii) ANMELDETITEL: Verfahren zur Diagnostik von Krankheiten mittels Genanalyse
   (iii) ANZAHL DER SEQUENZEN: 2
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 1517 Basenpaare
      (B) ART: Nukleinsäure
      (C) STRANGFORM: Doppel
      (D) TOPOLOGIE: linear
   (ii) ART DES MOLEKÜLS: cDNS zu mRNS
   (iii) HYPOTHETISCH: NEIN
   (iii) ANTISENSE: NEIN
   (vi) URSPRÜNLICHE HERKUNFT:
      (A) ORGANISMUS: Homo sapiens
   (ix) MERKMALE:
      (A) NAME/SCHLÜSSEL: CDS
      (B) LAGE: 1..1024
   (xi) SEOUENZBESCHREIBUNG: SEO ID NO: 1: 
ATG GGG GAG ATG GAG CAA CTG CGT CAG GAA GCG GAG CAG CTC AAG AAG 48

1               5                   10                  15
CAG ATT GCA GAT GCC AGG AAA GCC TGT GCT GAC GTT ACT CTG GCA GAG 96

            20                  25                  30
CTG GTG TCT GGC CTA GAG GTG GTG GGA CGA GTC CAG ATG CGG ACG CGG 144

        35                  40                  45
CGG ACG TTA AGG GGA CAC CTG GCC AAG ATT TAC GCC ATG CAC TGG GCC 192

    50                  55                  60
ACT GAT TCT AAG CTG CTG GTA AGT GCC TCG CAA GAT GGG AAG CTG ATC 240

65                  70                  75                  80
GTG TGG GAC AGC TAC ACC ACC AAC AAG GTG CAC GCC ATC CCA CTG CGC 288

                85                  90                  95
TCC TCC TGG GTC ATG ACC TGT GCC TAT GCC CCA TCA GGG AAC TTT GTG 336

 
100                 105                 110
GCA TGT GGG GGG CTG GAC AAC ATG TGT TCC ATC TAC AAC CTC AAA TCC     384

        115                 120                 125
CGT GAG GGC AAT GTC AAG GTC AGC CGG GAG CTT TCT GCT CAC ACA GGT     432

    130                 135                 140
TAT CTC TCC TGC TGC CGC TTC CTG GAT GAC AAC AAT ATT GTG ACC AGC     480

145                 150                 155                 160
TCG GGG GAC ACC ACG TGT GCC TTG TGG GAC ATT GAG ACT GGG CAG CAG     528

                165                 170                 175
AAG ACT GTA TTT GTG GGA CAC ACG GGT GAC TGC ATG AGC CTG GCT GTG     576

            180                 185                 190
TCT CCT GAC TTC AAT CTC TTC ATT TCG GGG GCC TGT GAT GCC AGT GCC     624

        195                 200                 205
AAG CTC TGG GAT GTG CGA GAG GGG ACC TGC CGT CAG ACT TTC ACT GGC     672

    210                 215                 220
CAC GAG TCG GAC ATC AAC GCC ATC TGT TTC TTC CCC AAT GGA GAG GCC     720

225                 230                 235                 240
ATC TGC ACG GGC TCG GAT GAC GCT TCC TGC CGC TTG TTT GAC CTG CGG     768

                245                 250                 255
GCA GAC CAG GAG CTG ATC TGC TTC TCC CAC GAG AGC ATC ATC TGC GGC     816

            260                 265                 270
ATC ACG TCT GTG GCC TTC TCC CTC AGT GGC CGC CTA CTA TTC GCT GGC     864

        275                 280                 285
TAC GAC GAC TTC AAC TGC AAT GTC TGG GAC TCC ATG AAG TCT GAG CGT     912

    290                 295                 300
GTG GGC ATC CTC TCT GGC CAC GAT AAC AGG GTG AGC TGC CTG GGA GTC     960

305                 310                 315                 320
ACA GCT GAC GGG ATG GCT GTG GCC ACA GGT TCC TGG GAC AGC TTC CTC    1008

                325                 330                 335
AAA ATC TGG AAC TGA G GAGGCTGGAG AAAGGGAAGT GGAAGGCAGT GAACACACTC    1064
  *
            340
AGCAGCCCCC TGCCCGACCC CATCTCATTC AGGTGTTCTC TTCTATATTC CGGGTGCCAT    1124
TCCCACTAAG CTTTCTCCTT TGAGGGCAGT GGGGAGCATG GGACTGTGCC TTTGGGAGGC    1184
AGCATCAGGG ACACAGGGGC AAAGAACTGC CCCATCTCCT CCCATGGCCT TCCCTCCCCA    1244
CAGTCCTCAC AGCCTCTCCC TTAATGAGCA AGGACAACCT GCCCCTCCCC AGCCCTTTGC    1304
AGGCCCAGCA GACTTGAGTC TGAGGCCCCA GGCCCTAGGA TTCCTCCCCC AGAGCCACTA    1364
CCTTTGTCCA GGCCTGGGTG GTATAGGGCG TTTGGCCCTG TGACTATGGC TCTGGCACCA    1424
CTAGGGTCCT GGCCCTCTTC TTATTCATGC TTTCTCCTTT TTCTACCTTT TTTTCTCTCC    1484
TAAGACACCT GCAATAAAGT GTAGCACCCT GGT                                 1517
(2) INFORMATION ZU SEQ ID NO: 1:
   (i) SEQUENZ CHARAKTERISTIKA:
      (A) LÄNGE: 341 Aminosäuren
      (B) ART: Aminosäure
      (D) TOPOLOGIE: linear
   ART DES MOLEKÜLS: Protein
   SEQUENZBESCHREIBUNG: SEQ ID NO: 2:

(i) SEQUENZ CHARAKTERISTIKA:
(A) LÄNGE: 341 Aminosäuren
(B) ART: Aminosäure
(D) TOPOLOGIE: linear
(ii) ART DES MOLEKÜLS: Protein
(xi) SEQUENZBESCHREIBUNG: SEQ ID NO: 2:

1               5                   10                  15

            20                  25                  30

        35                  40                  45

    50                  55                  60

65                  70                  75                  80

                85                  90                  95

            100                 105                 110

        115                 120                 125

    130                 135                 140

145                 150                 155                 160

                165                 170                 175

            180                 185                 190

        195                 200                 205

    210                 215                 220

225                 230                 235                 240

                245                 250                 255

            260                 265                 270

        275                 280                 285

    290                 295                 300

305                 310                 315                 320

                325                 330                 335
  *
            340

## Beschreibung

Die vorliegende Erfindung betrifft ein Verfahren zur Diagnostik von Erkrankungen mittels Genanalyse, insbesondere der Analyse von Genen für Untereinheiten der humanen Guaninnukleotid-bindenden Proteine (G-Proteine).

Heterotrimere Guaninnukleotid-bindende Proteine (G-Proteine) haben eine herausragende Bedeutung bei der intrazellulären Signaltransduktion. Sie vermitteln die Weiterleitung extrazellulärer Signale nach Stimulation von Hormonrezeptoren und anderen Rezeptoren, welche nach Rezeptoraktivierung eine Konformationsänderung durchmachen. Dies führt zur Aktivierung von G-Proteinen, welche nachfolgend intrazelluläre Effektoren (z.B. Ionenkanäle, Enzyme) aktivieren oder hemmen können. Heterotrimere G-Proteine sind aus drei Untereinheiten, den α-, β- und γ-Untereinheiten zusammengesetzt. Bislang wurden mehrere unterschiedliche α-Untereinheiten, 5 β-Untereinheiten und ca. 12 γ-Untereinheiten mittels biochemischer und molekularbiologischer Methoden nachgewiesen (Birnbaumer, L. and Birnbaumer, M. Signal transduction by G proteins: 1994 edition. *J.Recept.Res.* 15:213-252, 1995; Offermanns, S. and Schultz, G. Complex information processing by the transmembrane signaling system involving G proteins. *Naunyn Schmiedebergs Arch.Pharmacol.* 350:329-338, 1994; Nürnberg, B., Gudermann, T., and Schultz, G. Receptors and G proteins as primary components of transmembrane signal transduction. Part 2. G proteins: structure and function. *J.Mol.Med.* 73:123-132, 1995; Neer, E.J. Heterotrimeric G proteins: Organizers of Transmembrane Signals. Cell 80:249-257, 1995; Rens-Domiano, S. and Hamm, H.E. Structural and functional relationships of heterotrimeric G-proteins. FASEB J. 9:1059-1066, 1995).

Die rezeptorvermittelte Aktivierung bestimmter -Untereinheiten kann durch Vorbehandlung mit Pertussistoxin (PTX) gehemmt werden. Dazu gehören insbesondere die α-Isoformen αi1, αi2 und αi3, sowie unterschiedliche o -Untereinheiten. Solche G-Proteine werden auch als "PTX-sensitive G-Proteine" bezeichnet.

Es wurde gefunden, daß sich eine Genveränderung im Gen für humanes G-Protein β3-Untereinheit zur Diagnostik von Erkrankungen eignet. Diese Genveränderung eignet sich insbesondere zur Ermittlung des Risikos, an einer Krankheit, die mit G-Protein-Fehlsteuerung assoziiert ist, zu erkranken.

Gegenstand der Erfindung ist ein Verfahren gemäß Patentanspruch 1.

Die gefundene Genveränderung befindet sich im Gen für humanes G-Protein β3-Untereinheit. Dieses Gen ist von Levine et al. (Proc. Natl. Acad. Sci USA, Vol 87, Seite 2329-2333, (1990) beschrieben worden. Der codierende Bereich hat an Position 275 ein Ser-Codon (TCC), während Probanden mit erhöhtem Risiko für eine Erkrankung, die mit G-Protein-Fehlsteuerung assoziiert ist,an dieser Position das ebenfalls für Ser codierende Codon TCT besitzen. Die Genveränderung ist eine Basensubstitution an Position 825, bei der ein Cytosin (C) durch Thymin (T) ersetzt ist. Auf Aminosäureebene ist dieser Basenaustausch jedoch "stumm", d.h. er führt nicht zum Einbau einer anderen Aminosäure an dieser Position. Die bei Probanden mit erhöhtem Erkrankungsrisiko gefundene Sequenz ist in SEQ ID NO:1 im Sequenzprotokoll dargestellt.

Die gefundene Genveränderung tritt in der Regel in heterozygoter Form auf.

Unter Krankheiten, die mit einer Substitution von Cytosin durch Thymin an Position 825 in SEQ ID NO:1, also mit einer G-Protein Fehlsteuerung, assoziiert sind, sind solche Erkrankungen zu verstehen, bei denen das G-Protein in der Signaltransduktion involviert ist und seine Funktion nicht in physiologischer Weise erfüllt.

Die Fehlsteuerung kann eine Reihe von Ursachen haben, beispielsweise eine Veränderung im Strukturgen oder eine veränderte Genexpression.

Bei den Erkrankungen handelt es sich u.a. um Herz-Kreislauf Erkrankungen, Stoffwechselstörungen und Immunerkrankungen.

Als Herz-Kreislauf Erkrankungen sind zu nennen:

Hypertonie, Schwangerschaftshypertonie (Gestose, "hypertension in pregnancy"), koronare Herzkrankheit, lokalisierte und/oder generalisierte Atherosklerose, Stenosen der Blutgefäße, Restenose nach revaskularisierenden Gefäßeingriffen (z.B. PTCA mit und ohne Stentimplantation), Apoplexneigung. Thromboseneigung und gesteigerte Thrombozytenaggregation.

Als Stoffwechselstörungen sind zu nennen:

Metabolisches Syndrom, Insulinresistenz und Hyperinsulinämie, Typ II-Diabetes mellitus, diabetische Komplikationen (z.B. Nephropathie, Neuropathie, Retinopathie, etc.) Fettstoffwechselstörungen, gestörte zentrale Chemorezeption (C02-Toleranz, Azidosetoleranz, plötzlicher Kindstod (SIDS)).

Als Immunerkrankungen sind zu nennen:

Gestörte Stärke der körpereigenen Immunantwort (Bildung von Immunglobulinen, Aggressivität von T-Zellen und NK-Zellen), gestörte generelle Proliferationsneigung inkl. Wundheilungsvermögen, Neigung zur Tumorentstehung und Proliferation inkl. Metastasierungspotential maligne transformierter Zellen, Dauer der Latenzzeit nach HIV-Infektion bis zum klinischen Ausbruch der Erkrankung, Kaposi-Sarkom, Neigung zu Leberzhirrose, Transplantatoleranz und Transplantatabstoßung.

Die erfindungsgemäße Verwendung der Genmutation eignet sich besonders zur Ermittlung des Erkrankungsrisikos an Hypertonie.

Ein weiterer Gegenstand der Erfindung ist die Herstellung von transgenen Tieren, die die oben beschriebene Genmutation tragen. Solche transgenen Tiere sind vor allem als Tiermodelle für die Untersuchung und Therapie der oben beschriebenen Krankheiten von großer Bedeutung. Die Verfahren zur Erzeugung transgener Tiere sind dem Fachmann allgemein bekannt.

Bei dem erfindungsgemäßen Verfahren zur Ermittlung des relativen Erkrankungsrisikos wird einem Probanden Körpermaterial entnommen, das die genetische Information des Probanden enthält. Dies wird in der Regel durch Blutentnahme und Isolierung der Nukleinsäure hieraus erreicht.

Aus der isolierten Nukleinsäure des Probanden wird die Genstruktur für das G-Protein β3-Untereinheit ermittelt und mit der in SEQ ID NO:1 angegebenen Sequenz verglichen.

Die Ermittlung der Genstruktur kann durch Sequenzierung der Nukleinsäure erfolgen. Dies kann entweder direkt aus der genomischen DNA oder nach Amplifizierung der Nukleinsäure beispielsweise mittels PCR-Technik erfolgen.

Die Genstruktur kann auf genomischer Ebene oder auch auf mRNA oder cDNA Ebene erfolgen.

Bevorzugt ist die Ermittlung durch Sequenzierung nach PCR-Amplifikation der cDNA. Die für die PCR-Reaktion geeigneten Primer lassen sich für den Fachmann leicht aus den in SEQ ID N0:1 dargestellten Sequenzen ableiten. Man verfährt dabei vorteilhafterweise so, daß jeweils ein Strang und Gegenstrang bindender Primer vor und nach der relevanten Basenposition 825 gewählt wird.

Der Genvergleich kann jedoch auch mit anderen Methoden, beispielsweise durch selektive Hybridisierung oder durch entsprechende Kartierung mit Restriktionsenzymen durchgeführt werden. Der Basenaustausch C→T an der oben beschriebenen Position 825 führt zum Verlust einer Schnittstelle für das Restriktionsenzym Dsa I, was ebenfalls dem Nachweis dieses genetischen Polymorphismus dient.

Wenn der Proband an der Position 825 ein Thymin (T) trägt, ist ihm ein höheres Erkrankungsrisiko zuzuordnen als einem Probanden mit einem Cytosin (C) an dieser Position.

Die Erfindung ist in den folgenden Beispielen weiter veranschaulicht.

### Beispiel 1

Nachweis der Genveränderung bei Hypertonikern durch Sequenzierung

In Voruntersuchungen konnte eine gesteigerte Aktivierbarkeit PTX-sensitiver G-Proteine bei Patienten mit essentieller Hypertonie nachgewiesen werden. Dieser Nachweis gelang in immortalisierten Zellen solcher Patienten, die als phänotypischen Marker eine gesteigerte Aktivität des Na/H- Austauschers aufweisen. Die gesteigerte Aktivierbarkeit PTX-sensitiver G-Proteine hat wichtige Konsequenzen für die Zellfunktion. Dazu gehören eine gesteigerte Bildung intrazellulärer "second messenger" - Moleküle (z.B. Inositol-1,4,5-trisphosphat), eine gesteigerte Freisetzung intrazellulärer Ca²⁺-Ionen, eine vermehrte Bildung von Immunglobulinen und ein beschleunigtes Zellwachstum. Da diese Veränderungen in immortalisierten Zellen und nach langer Zellkulturdauer nachzuweisen sind, kann man davon ausgehen, daß diese Veränderung genetisch fixiert ist (Rosskopf, D., Frömter, E., and Siffert, W. Hypertensive sodium-proton exchanger phenotype persists in immortalized lymphoblasts from essential hypertensive patients-a cell culture model for human hypertension. J.Clin.Invest. 92:2553-2559, 1993; Rosskopf, D., Hartung, K., Hense, J., and Siffert, W. Enhanced immunoglobulin formation of immortalized B cells from hypertensive patients. Hypertension 26:432-435, 1995; Rosskopf, D., Schröder, K.-J., and Siffert, W. Role of sodium-hydrogen exchange in the proliferation of immortalised lymphoblasts from patients with essential hypertension and normotensive subjects. Cardiovasc.Res. 29:254-259, 1995; Siffert, W., Rosskopf, D., Moritz, A., Wieland, T., Kaldenberg-Stasch, S., Kettler, N., Hartung, K., Beckmann, S., and Jakobs, K.H. Enhanced G protein activation in immortalized lymphoblasts from patients with essential hypertension. J.Clin.Invest. 96:759-766, 1995.

Aus immortalisierten Zellinien von Hypertonikern wurde nach Standardverfahren RNS präpariert und mittels der reversen Transkriptase in cDNS umgeschrieben. Mittels Polymerase-Kettenreaktion (PCR) wurde die für die G-Proteinuntereinheit G3 kodierende cDNS amplifiziert und sequenziert. Für die PCR-Reaktion wurden die folgenden Oligonukleotid-Primer eingesetzt: 5'-TGG GGG AGA TGG AGC AAC TG und

5'-CTG CTG AGT GTG TTC ACT GCC.

Im Vergleich zu der von Levine et al. publizierten Sequenz (Levine, M.A., Smallwood, P.M. , Moen, P.T., Jr., Helman, L.J., and Ahn, T.G. Molecular cloning of β3 subunit, a third form of the G Protein β-subunit polypeptide. Proc. Natl. Acad. Sci. USA 87(6):2329-2333, 1990) wurde in der cDNS aus Hypertonikerzellen die folgende Abweichung gefunden: Nukleotid 825 Cytosin (C) im Bereich der kodierenden Sequenz wird durch ein Trymin (T) ersetzt (Nukleotid 1 entspricht der Base A des Startcodons ATG). Dieser Basenaustausch führt zu einem stummen Polymorphismus,d.h. die durch das entsprechende Basentriplett kodierte Aminosäure (Serin) wird gegenüber der Originalsequenz nicht verändert. Die gefundene DANN-Sequenz ist in SEQ ID NO:1 beschrieben.

### Beispiel 2

### Nachweis der Genveränderung bei Hypertonikern durch Restriktionsenzym-Analyse

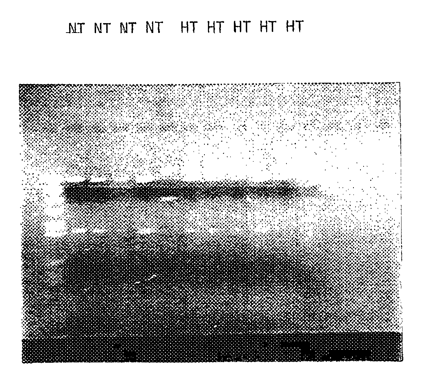

In der Abbildung ist ein Genvergleich von Normotonikern und Hypertonikern durch Restriktionsenzymanalyse dargestellt. Hier wurde die mittels PCR amplifizierte, für G3 kodierende cDNS aus Zellen von Normotonikern (NT) und Hypertonikern (HT) einer Restriktionsenzymanalyse mit dem Enzym Dsa I unterzogen. Die Reaktionsprodukte wurden in einem Agarosegel aufgetrennt, was in der Abbildung dargestellt ist.

Man erkennt in der Abbildung deutlich die vollständige Restriktion der G3 cDNS aus Normotonikerzellen nach Verdau mit Dsa I. Die cDNS aus Hypertonikerzellen wird nur teilweise durch Dsa I geschnitten. Neben den zu erwartenden Schnittprodukten ergibt sich zudem ungeschnittenes PCR-Produkt. Links und rechts sind Referenzfragmente (Marker) zum Größenvergleich aufgetragen. Vier von fünf der hier dargestellten DNA-Sequenzen von Hypertonikern zeigen den oben beschriebenen Basenaustausch und sind für diese Veränderung heterozygot.

## Patentansprüche

1. Verfahren zur Ermittlung des Risikos zur Erkrankung an einer Krankheit, die mit einer Substitution von Cytosin durch Thymin an Position 825 in SEQ ID NO:1 assoziiert ist, bei welchem eine Genveränderung im Gen für humanes G-Protein β3-Untereinheit identifiziert wird.

2. Verwendung nach Anspruch 1, **dadurch gekennzeichnet, daß** die Krankheit eine Herz-Kreislauf-Erkrankung, eine Stoffwechselstörung oder eine Immunerkrankung ist.

3. Verwendung nach Anspruch 1, **dadurch gekennzeichnet, daß** die Krankheit Hypertonie ist.

4. Verfahren nach einem der Ansprüche 1 bis 3, **dadurch gekennzeichnet, daß** die Genveränderung durch Sequenzierung identifiziert wird.

5. Verfahren nach Anspruch 4, **dadurch gekennzeichnet, daß** vor der Sequenzierung ein Genabschnitt, der Position 825 beinhaltet, amplifiziert wird.

6. Verfahren nach einem der Ansprüche 1 bis 3, **dadurch gekennzeichnet, daß** die Genveränderung durch Hybridisierung identifiziert wird.

7. Verfahren nach einem der Ansprüche 1 bis 3, **dadurch gekennzeichnet, daß** die Genveränderung durch Spaltung mittels Restriktionsenzymen identifiziert wird.

8. Verfahren nach Anspruch 7, **dadurch gekennzeichnet, daß** das Restriktionsenzym Dsa I verwendet wird.

## Claims

1. A method for establishing the risk of developing a disorder associated with a substitution of cytosine by thymine at position 825 in SEQ ID No:1, wherein a genetic modification is identified in the gene for human G protein β3 subunit.

2. Use according to claim 1, wherein the disorder is a cardiovascular disease, a metabolic disturbance or an immunological disease.

3. Use according to claim 1, wherein the disorder is hypertension.

4. A method according to claims 1 to 3, wherein the genetic modification is identified by sequencing.

5. A method according to claim 4, wherein a gene section which includes position 825 is amplified before sequencing.

6. A method according to claims 1 to 3, wherein the genetic modification is identified by hybridization.

7. A method according to claims 1 to 3, wherein the genetic modification is identified by cleavage using restriction enzymes.

8. A method according to claim 7, wherein the restriction enzyme Dsa I is used.

## Revendications

1. Procédé pour la détermination du risque de contracter une maladie qui est associée à une substitution de la cytosine par de la thymine en position 825 de la séquence SEQ ID N°1, dans lequel on identifie une modification génétique dans le gène codant pour la sous-unité β3 de la protéine G humaine.

2. Utilisation selon la revendication 1, **caractérisée en ce que** la maladie est une maladie cardio-vasculaire, un trouble métabolique ou une maladie du système immunitaire.

3. Utilisation selon la revendication 1, **caractérisée en ce que** la maladie est l'hypertonie.

4. Procédé selon l'une des revendications quelconques 1 à 3, **caractérisé en ce que** la modification génétique est identifiée par séquençage.

5. Procédé selon la revendication 4, **caractérisé en ce qu'**avant séquençage, on amplifie un segment du gène contenant la position 825.

6. Procédé selon Tune quelconque des revendications 1 à 3, **caractérisé en ce que** l'on identifie la modification génétique par hybridation.

7. Procédé selon l'une quelconque des revendications 1 à 3, **caractérisé en ce que** l'on identifie la modification génétique par scission au moyen d'une enzyme de restriction.

8. Procédé selon la revendication 7 **caractérisée en ce que** l'on utilise Dsa I en tant qu'enzyme de restriction.
